# FASCICULE DE BREVET EUROPEEN

(11) **EP 0 906 075 B1**
(45) Date de publication et mention de la délivrance du brevet: **13.09.2000**
(21) Numéro de dépôt: 97926049.4
(22) Date de dépôt: 28.05.1997
(51) Int. Cl.: A61K 6/00, A61K 6/083, C08J 7/12

(54) **PROTHESES DENTAIRES MODIFIEES EN SURFACE ET PROCEDE D'OBTENTION**
OBERFLÄCHENMODIFIZIERTE DENTALPROTHESEN UND VERFAHREN ZU IHRER HERSTELLUNG
DENTAL PROSTHESES WITH MODIFIED SURFACE AND METHOD OF PRODUCTION.

(30) Priorité: 30.05.1996 FR 9606680
(43) Date de publication de la demande: 07.04.1999
(73) Titulaire: Bellesort, Stephane, 72000 Le Mans (FR)
(72) Inventeur: Bellesort, Stephane, 72000 Le Mans (FR)
(74) Mandataire: Phélip, Bruno
(86) Numéro de dépôt international: FR9700932
(87) Numéro de publication internationale: WO9745092

(56) Documents cités:
- EP-A- 0 096 573
- EP-A- 0 373 385
- EP-A- 0 401 385
- EP-A- 0 487 418
- FR-A- 2 111 959
- US-A- 4 504 228

## Description

La présente invention est relative à des prothèses dentaires modifiées en surface.

Elle est en outre relative à un procédé d'obtention de ces prothèses.

La France compte 10 millions de porteurs de prothèses dentaires adjointes. 38% sont porteurs de prothèses adjointes complètes, 57% sont porteurs de prothèses adjointes partielles et 5% sont porteurs des deux types de prothèses.

En extrapolant à la Communauté Européenne, le nombre de porteurs de prothèses adjointes se situe vraisembablement aux alentours de 60 millions d'individus.

Les prothèses dentaires en résine, en particulier en polyméthacrylate, qu'elles soient partielles ou complètes ont une énergie de surface intermédiaire, comprise entre 35 et 45 mJ.m⁻². Du fait de cette énergie, et de la porosité du matériau, ces prothèses présentent deux inconvénients majeurs:
- une adhésion importante des protéines qui provoque : candidoses, caries, et stomatites prothétiques, et
- une rétention insuffisante des appareils complets et principalement des appareils complets inférieurs.

Divers moyens ont été employés pour améliorer la rétention de ces prothèses. Ainsi, dès le XVlème siècle, des ressorts reliant les appareils maxillaires et mandibulaires étaient posés sur les prothèses. Puis au XVIIIème siècle les prothèses étaient traitées dans une chambre à vide de Fauchard. Enfin, des aimants répulsifs ont été installés sur certaines prothèses.

Plus récemment, on a cherché à améliorer les prothèses en augmentant l'énergie de surface des matériaux les composant. Ainsi, des résines hydrophiles ont été fabriquées.

On a aussi cherché à recouvrir des polyméthacrylates de méthyle par du dioxyde de silicium. Néanmoins, ce procédé s'est révélé onéreux et les prothèses en résultant étaient instables.

La demande EP-0 401 385 (Mitsubishi Rayon Co., Ltd), quant à elle, décrit le traitement de prothèses en résines acryliques par un halogène ou un composé halogéné, préférentiellement à base d'iode. Ce traitement présente les inconvénients de modifier la biocompatibilité des résines et d'augmenter les volumes des prothèses. En outre, ce traitement ne permet que d'éviter la formation de la plaque dentaire, et n'agit pas sur la rétention des prothèses. Enfin, l'iode qui est le composé préférentiellement utilisé est allergisant.

Les intrados prothétiques des prothèses dentaires complètes ont aussi été soumis à un sablage au quartz pendant 30 à 60 secondes. Néanmoins, cette technique s'est révélée elle aussi inadaptée, du fait de l'augmentation considérable de la colonisation bactérienne.

Des adhésifs dentaires sont aussi utilisés afin d'assurer le maintien en bouche des prothèses dentaires. Néanmoins, ces adhésifs nécessitent d'être utilisés une ou plusieurs fois par jour. De plus, ils représentent un coût important.

Les prothèses dentaires en résine sont en outre responsables du développement d'une plaque dentaire importante. Ainsi, les germes Gram négatifs sont trois fois plus nombreux chez un porteur d'une prothèse neuve, par rapport à un individu sain et denté, et onze fois plus nombreux chez les porteurs de prothèses anciennes. Les entérobactéries sont sept fois plus nombreuses chez le porteur de prothèses neuves, par rapport à l'individu sain et denté. Les Candidas quant à elles sont huit fois plus nombreuses chez le porteur de prothèses neuves et vingt sept fois plus nombreuses chez le porteur de prothèses anciennes.

Cette plaque bactérienne entraîne une acidose prothétique responsable de caries, de gingivites, de candidoses, et de stomatites sous-prothétiques chez le porteur de prothèses en résine.

Afin d'éviter le développement de cette plaque dentaire, les porteurs doivent respecter une hygiène rigoureuse et utiliser des produits d'entretien mais aussi ne doivent pas porter les prothèses la nuit, ce qui est généralement mal accepté par les porteurs.

Il n'existait donc pas de moyens permettant une rétention efficace des prothèses, et une limitation à un niveau acceptable du développement de la plaque bactérienne.

Le traitement de divers objets par des plasmas froids est bien connu.

Ainsi, la demande FR 2 620 624 (IRAP, CARDIAL, DE LA FAYE) décrit des objets divers destinés à être mis en contact avec du sang, tels que des cathéters, des seringues, ou des parties de prothèses vasculaires ou valvulaires cardiaques, constitués de polymères dont les atomes d'hydrogène présents à leur surface exposée ont été remplacés par des atomes de fluor ou des groupements CF₂ ou CF₃. Les polymères sont en particulier le polyéthylène, le poly(chlorure de vinyle) ou le poly(éthylène téréphtalate). Les atomes d'hydrogène sont remplacés de telle sorte que le fluor total représente au plus 10% des atomes présents à leur surface. Les objets sont traités dans des plasmas fluorés pendant une durée allant de quelques secondes à une dizaine de minutes, à une puissance allant de 0,1 watt à 2 watts par litre de capacité de réacteur, et sous une pression de l'ordre de 10 à 10.000 Pa.

La demande EP-0 487.418 (IRAP, Groupe FIDOMI) décrit quant à elle des dispositifs à usage ophtalmologique formés de substrats polymériques dont les atomes d'hydrogène présents à leur surface ont été remplacés par des atomes de fluor ou des groupements CF, CF₂ ou CF₃. Le fluor total représente au moins 15% des atomes présents sur cette surface. Les polymères peuvent être du polyméthacrylate de méthyle (PPMA), un polymère de 2-hydroxy-éthylméthacrylate (HEMA) du silicone ou un polysulfonate. Les dispositifs sont traités dans des gaz fluorés appropriés durant 1 à 20 minutes, à une puissance d'émission du réacteur comprise entre 3 et 10 watts par litre de capacité de réacteur, et sous une pression négative comprise entre 0,1 et 1 torr.

DEJUN LI et JIE ZHAO (1995, J. Adhesion Sci. Technol., Vol.9, pp 1249-1261), décrivent le traitement d'objets en polyuréthane par des plasmas froids d'argon et d'azote, durant de 1 à 15 minutes, à une puissance de 100 watts, et à une pression de 0,3 à 15 Pa. Les auteurs montrent que ces traitements induisent des diminutions de l'angle de contact de l'eau et une augmentation du temps de coagulation. Les applications de ces traitements sont donc à l'évidence destinées à des objets ou dispositifs devant être au contact du sang.

La demande EP-0 096 573 (The United States of America) décrit le traitement de fibres textiles par des plasmas froids. Aucune application aux prothèses n'est mentionnée.

GEBHARDT et al. (1995, Proceedings , 12th International Symposium on Plasma Chemistry, Minneapolis, Minnesota, USA, Vol.1, 155-160) décrivent le traitement de feuilles de polyéthylène à haute densité (HDPE) par un plasma d'argon puis le greffage de groupements styrène et de groupements 2-(chloroéthyl)vinyl éther, par exposition à des vapeurs. Il ne s'agit donc pas dans cet article d'un traitement simple par des plasmas froids, mais d'un traitement combinant une étape de traitement par des plasmas froids et une étape de traitement chimique. Les auteurs montrent qu'une telle surface présente une meilleure biocompatibilité vis-à-vis d'hépathocytes de rat.

Il ressort de l'analyse de l'état de la technique que le traitement de prothèses dentaires par des plasmas froids n'a jamais été décrit.

Or, les tissus oculaires, ou les cellules sanguines, présentent des différences considérables avec les tissus de la cavité buccale. Ainsi, la cavité buccale comprend un nombre bien plus élevé de type de tissus cellulaires par rapport à la cavité oculaire. Les caractéristiques des polymères au contact de ces différents tissus et types cellulaires sont de ce fait différentes, et non transposables d'un organe à l'autre.

Le demandeur s'est donc attaché à déterminer des conditions de traitement des polymères constituant les prothèses dentaires, permettant d'améliorer leur rétention et/ou de limiter le développement de la plaque dentaire.

Il a montré que le traitement de ces polymères par des plasmas froids permettait de résoudre ces problèmes.

La présente invention est donc relative à des prothèses dentaires composées principalement d'un polymère contenant des atomes d'hydrogène et ayant les qualités mécaniques et chimiques requises, caractérisées en ce que les atomes d'hydrogène à la surface dudit polymère ont été remplacés par des atomes de fluor, des groupements -CF, -CF₂,-CF₃, -OH,- CO₂H, -C=O,- OOH, -NH₂,- C=NH et/ou -CONH₂.

Selon un premier mode de mise en oeuvre, les prothèses sont des appareils complets, auquel cas les atomes d'hydrogène dudit polymère sont préférentiellement remplacés par les groupements -OH, CO₂H, -C=O, - OOH, NH2, -C=NH et/ou -CONH2.. De manière préférentielle, lesdits polymères sont caractérisés en ce qu'ils présentent une teneur en oxygène sur une épaisseur de 30 nm à la surface de la prothèse , augmentée de 1 à 15%, et encore plus préférentiellement de 1 à 10%.

De tels polymères sont particulièrement bien adaptés aux appareils complets car ils présentent une énergie de surface augmentée par rapport au polymère non traité et de ce fait permettent d'obtenir une rétention améliorée de la prothèse et une moindre colonisation bactérienne. Des prothèses présentant de telles caractéristiques peuvent être obtenues par traitement, dans leur forme appropriée à leur utilisation, par un plasma d'argon, d'azote, de gaz carbonique, ou d'oxygène, à une puissance d'émission comprise entre 1 et 10 watts, et préférentiellement 2 et 5 watts par litre de capacité du réacteur.

Les traitements à l'argon ou à l'azote sont plus particulièrement adaptés, car ils évitent la formation de groupements à oxydation rapide à la surface des prothèses.

Selon un second mode de mise en oeuvre de l'invention, les atomes d'hydrogène à la surface du polymère constituant la prothèse sont remplacés majoritairement par des atomes de fluor, ou des groupements CF, CF₂ ou CF₃. De manière préférentielle, de 10 à 60%, préférentiellement de 20 à 50%, des atomes d'hydrogène présents sur une épaisseur de 30 nm à la surface dudit polymère, ont été remplacés par des atomes de fluor, des groupements CF, CF₂ ou CF₃.

Des prothèses à base des polymères selon ce second mode de mise en oeuvre de l'invention présentent une énergie de surface diminuée et de ce fait sont plus particulièrement adaptées aux appareils partiels. En effet, dans ce type d'appareils il n'est pas indispensable d'obtenir une rétention améliorée par traitement de surface, car la rétention est mécanique: crochets, attachements, boutons, pression. En tout état de cause, de tels polymères limitent la formation de la plaque dentaire, en raison de la faible énergie de surface.

Les prothèses selon le second mode de mise en oeuvre de l'invention peuvent être obtenues par un procédé dans lequel elle sont traitées, dans leur forme appropriée à leur utilisation, par un plasma de molécule fluorée non polymérisable dans un réacteur avec une puissance d'émission comprise entre 1 et 5 watts par litre de capacité de réacteur.

Quel que soit le procédé mis en oeuvre, le temps de traitement dans le réacteur est avantageusement compris entre 2 et 20 minutes, préférentiellement entre 5 et 15 minutes. La pression quant à elle se situe avantageusement entre 0,01, 10 Torr et préférentiellement entre 0,1 et 1 Torr.

De manière avantageuse, le polymère constituant les prothèses est un polyméthacrylate d'éthyle, un polyméthacrylate de méthyle (PMMA), éventuellement réticulé, ou un mélange de ces deux polymères, ou d'autres dérivés acryliques. Il peut être aussi un polymère à base de polydiméthylsiloxane.

Il peut aussi être tout autre polymère ayant des qualités mécaniques et chimiques compatibles avec la fabrication de prothèses dentaires, et présentant des atomes d'hydrogène susceptibles d'être substitués par d'autres atomes groupements ou fonctions par un traitement à l'aide de plasmas froids.

Ces traitements permettent donc de résoudre les problèmes qui se posaient pour la fabrication de prothèses dentaires. Ils permettent de garantir les qualités intrinsèques de ces prothèses tant chimiques que mécaniques. En effet, de tels procédés- ne modifient pas la géométrie de ces prothèses. En outre, ils ne nécessitent pas l'utilisation de solvant ou de réactif, ce qui évite toute contamination chimique des prothèses.

Enfin, ils sont parfaitement stables et reproductibles, ce qui constitue un gage de qualité.

Les traitements par les plasmas froids sont effectués selon des méthodes connues de l'homme du métier. A cet effet, il pourra se référer au manuel général suivant : « Plasma Réactifs; A. Ricard, 1995, Editions de la Société Française du Vide ».

La présente invention est illustrée, sans pour autant être limitée, par les exemples qui suivent:

### EXEMPLE 1:

### Traitement de prothèses en polyméthacrylate de méthyle par des plasmas non-fluorés.

L'appareil dentaire en polyméthacrylate de méthyle est préalablement nettoyé dans une solution d'hexethidine ou de chlorhexidine.

Il est ensuite posé sur une plaque dans un réacteur à plasma à type capacitif.

Le vide est alors crée à l'aide d'une pompe, jusqu'à obtenir une dépression de 0,1 Torr.

Le réacteur présente un volume de 15 litres, et émet à une longueur d'onde de 13,56 Mhz.

Les prothèses sont traitées durant 10 minutes, à une puissance de 50 watts, respectivement soit dans de l'oxygène, du gaz carbonique, de l'argon ou de l'azote. L'analyse de la surface des prothèses sur une couche d'une épaisseur de 30 nm montre que la teneur en oxygène augmente de l'ordre de 1 à 10%, dans le cas d'un traitement par de l'argon.

Dans le cas d'un traitement par de l'azote, la teneur en oxygène est augmentée d'une manière similaire, et la teneur en azote est augmentée de 1 à 5%.

Les angles de contact de l'eau par rapport au PPMA non traité, et traité par les quatre différents plasmas, ont été mesurés. Les résultats sont indiqués dans le tableau.

Les résultats obtenus sont remarquables, car, comme le montre le tableau, l'angle de contact est pratiquement divisé par deux, ce qui signifie que l'énergie de surface du polymère ainsi traité est fortement augmentée. En effet, l'énergie suit une loi du type: énergie = F(1 + cosθ), dans laquelle θ représente l'angle de contact.

Les prothèses ainsi obtenues présentent une rétention améliorée et un développement limité de la plaque dentaire au niveau de l'intrados prothétique.

### EXEMPLE 2:

### Traitement de prothèse par des plasmas fluorés

Les traitements ont été effectués sur des appareils partiels, à l'aide de gaz fluorés CF₄ et SF₆, dans les conditions indiquées dans l'exemple 1.

Les résultats obtenus montrent que ce type de traitement, contrairement aux traitements illustrés par l'exemple 1, augmentent l'angle de contact de l'eau avec le polymère et donc diminuent l'énergie de surface.

Une telle diminution de l'énergie de surface diminue la rétention des prothèses, mais, comme dans l'exemple 1, limite l'adhésion de la plaque bactérienne au niveau de l'intrados prothétique.

**TABLEAU**

| Témoin 1 | Témoin 2 | Témoin 3 | Témoin 4 |
|---|---|---|---|
| 77° | 74° | 84° | 78° |
| (écart 7° sur 6 mesures) | (écart 10° sur 6 mesures) | (écart 5° sur 6 mesures) | (écart 12° sur 6 mesures) |

| O₂ | CO₂ | Ar | N₂ |
|---|---|---|---|
| 39° | 43° | 42° | 42° |
| (écart 14° sur 6 mesures) | (écart 17° sur 6 mesures) | (écart 14° sur 6 mesures) | (écart 12° sur 6 mesures) |

## Revendications

1. Prothèse dentaire composée d'un polymère contenant des atomes d'hydrogène et ayant les qualités mécaniques et chimiques requises, caractérisée en ce que les atomes d'hydrogène à la surface dudit polymère sont remplacés partiellement par des atomes de fluor, des groupements - CF, -CF₂ -CF₃, -OH, -COOH, -C=O, -OOH, -NH₂, -C=NH ou -CONH₂.

2. Prothèse dentaire selon la revendication 1, caractérisée en ce que la teneur en oxygène, sur une épaisseur de 30 nm, à la surface dudit polymère, est augmentée de 1 à 15 %, préférentiellement de 1 à 10%.

3. Prothèse dentaire selon la revendication 1, caractérisée en ce que de 10 à 60%, préférentiellement de 20 à 50%, des atomes d'hydrogène sur une épaisseur de 30 nm à la surface dudit polymère sont remplacés par des atomes de fluor, des groupements CF, CF₂ ou CF₃.

4. Prothèse dentaire selon l'une des revendications 1 à 3, caractérisée en ce que le polymère est du polyméthacrylate de méthyle, du polyméthacrylate d'éthyle, ou un mélange de ces deux polymères et/ou d'autres dérivés acryliques.

5. Prothèse dentaire selon l'une des revendications 1 à 3, caractérisée en ce que ledit polymère est un polymère de polydiméthylsiloxane.

6. Procédé d'obtention de prothèses selon l'une des revendications 1, 2, 4 et 5, caractérisé en ce que lesdites prothèses sont traitées, dans la forme appropriée à leur utilisation, par un plasma d'oxygène, d'argon, de gaz carbonique ou d'azote, dans des réacteurs à une puissance d'émission comprise entre 1 et 10 watts/l de capacité du réacteur.

7. Procédé d'obtention de prothèses selon l'une des revendications 1, 3 , 4 et 5 caractérisé en ce que lesdites prothèses sont traitées, dans leur forme appropriée à leur utilisation, par un plasma de molécules fluorées non polymérisables, dans un réacteur à une puissance d'émission comprise entre 1 et 10 watts/l de capacité du réacteur.

8. Procédé selon la revendication 7 caractérisé en ce que ledit plasma est un gaz de CF₄ ou de SF₆.

9. Procédé d'obtention de prothèses selon l'une des revendications 6 et 7 caractérisé en ce que les prothèses sont traitées durant 2 à 20 minutes, préférentiellement de 5 à 15 minutes.

10. Prothèses susceptibles d'être obtenues par un procédé selon l'une des revendications 6 à 9.

## Patentansprüche

1. Zahnprothese, bestehend aus einem Polymer, das Wasserstoffatome enthält und die nötigen mechanischen und chemischen Eigenschaften aufweist, dadurch gekennzeichnet, dass die Wasserstoffatome an der Oberfläche des Polymers teilweise durch Fluoratome oder durch CF-, CF₂-, CF₃-, OH-, COOH-, C=O-, OOH-, NH₂-, C=NH- und/oder CONH₂-Gruppen ersetzt sind.

2. Zahnprothese nach Anspruch 1, dadurch gekennzeichnet, dass der Sauerstoffgehalt an der Oberfläche des Polymers über eine Dicke von 30 nm um 1 bis 15 %, vorzugsweise um 1 bis 10 %, angereichert ist.

3. Zahnprothese nach Anspruch 1, dadurch gekennzeichnet, dass 10 bis 60 %, vorzugsweise 20 bis 50 %, der Wasserstoffatome über eine Dicke von 30 nm an der Oberfläche des Polymers durch Fluoratome oder CF-, CF₂- oder CF₃-Gruppen ersetzt sind.

4. Zahnprothese nach einem der Ansprüche 1 bis 3, dadurch gekennzeichnet, dass das Polymer ein Methyl-Polymethacrylat, Ethyl-Polymethacrylat oder eine Mischung dieser beiden Polymere und/oder anderer Acrylderivate ist.

5. Zahnprothese nach einem der Ansprüche 1 bis 3, dadurch gekennzeichnet, dass das Polymer ein Polydimethylsiloxan ist.

6. Verfahren zur Herstellung von Prothesen nach einem der Ansprüche 1, 2, 4 und 5, dadurch gekennzeichnet, dass die Prothesen in einer mit ihrer Nutzung vereinbarenden Weise in Reaktoren mit einer Emissionsleistung von 1 bis 10 Watt/l Reaktorkapazität mit Sauerstoff-, Argon-, Kohlendioxid- oder Stickstoffplasma behandelt werden.

7. Verfahren zur Herstellung von Prothesen nach einem der Ansprüche 1, 3, 4 und 5, dadurch gekennzeichnet, dass die Prothesen in einer mit ihrer Nutzung vereinbarenden Weise in Reaktoren mit einer Emissionsleistung von 1 bis 10 Watt/l Reaktorkapazität mit einem Plasma aus nicht polymerisierenden Fluormolekülen behandelt werden.

8. Verfahren nach Anspruch 7, dadurch gekennzeichnet, dass das Plasma ein CF₄-Gas oder ein SF₆-Gas ist.

9. Verfahren zur Herstellung von Prothesen nach einem der Ansprüche 6 und 7, dadurch gekennzeichnet, dass die Prothesen während einer Dauer von 2 bis 20 Minuten, vorzugsweise von 5 bis 15 Minuten, behandelt werden.

10. Prothesen, die mit Hilfe eines Verfahrens nach einem der Ansprüche 6 bis 9 hergestellt sind.

## Claims

1. Dental prosthesis comprising a polymer containing hydrogen atoms and having the required mechanical and chemical properties, characterized in that the hydrogen atoms on the surface of the said polymer are partially replaced by fluorine atoms, CF, -CF₂, -CF₃, -OH, -COOH. -C=O, -OOH, -NH₂, -C=NH or CONH₂ groups.

2. Dental prosthesis according to claim 1, characterized in that the oxygen content over a thickness of 30 nm at the surface of the said polymer is increased by 1 to 15%, and preferably 1 to 10%.

3. Dental prosthesis according to claim 1, characterized in that 10 to 60%, and preferably 20 to 50%, of the hydrogen atoms over a thickness of 30 nm at the surface of the said polymer are replaced by fluorine atoms, CF, CF₂ or CF₃ groups.

4. Dental prosthesis according to one of claims 1 to 3, characterized in that the polymer is a polymethylmethacrylate, polyethylmethacrylate, or a mixture of these two polymers and/or other acrylic derivatives.

5. Dental prosthesis according to one of claims 1 to 3, characterized in that the said polymer is a polydimethylsiloxane polymer.

6. Process for manufacturing prostheses according to one of the claims 1, 2, 4 and 5, characterized in that the said prostheses are treated by an oxygen, argon, carbon dioxide or nitrogen plasma in a form appropriate for their use, in reaction vessels with an emission power of between 1 and 10 watts/l of capacity of the reaction vessel.

7. Process for manufacturing prostheses according to one of claims 1, 3, 4 and 5, characterized in that the said prostheses are treated by a plasma of molecules containing fluorine that cannot be polymerized in a form appropriate for their use, in a reaction vessel with an emission power of between 1 and 10 watts/l of capacity of the reaction vessel.

8. Process according to claim 7, characterized in that the said plasma is a CF₄ or SF₆ gas.

9. Process for manufacturing prostheses according to one of claims 6 and 7, characterized in that the prostheses are treated for 2 to 20 minutes, and preferably for 5 to 15 minutes.

10. Prostheses that may be manufactured by a process according to one of claims 6 to 9.
